# DEMANDE DE BREVET EUROPEEN

(11) **EP 0 951 870 A1**
(43) Date de publication de la demande: **27.10.1999**
(21) Numéro de dépôt: 98870089.4
(22) Date de dépôt: 21.04.1998
(51) Int. Cl.: A61B 17/12, A61B 17/34

(54) **Dispositif pour le traitement d'anévrisme**

(71) Demandeur: MEDICORP S.A., 54600 Villers-lès-Nancy (FR)
(72) Inventeur: La désignation de l'inventeur n'a pas encore été déposée
(74) Mandataire: Bairiot-Delcoux, Mariette

(57) **Abrégé**

Dispositif pour le traitement de lésions de la paroi de vaisseaux sanguins, en particulier les faux-anévrismes par coagulation, qui comprend un introducteur (6) à valve et à aiguille amovible (7), un filament (8, 18, 28, 38) apte à être introduit dans la poche (4) de cet anévrisme et à s'y enrouler sur lui-même et un moyen (19) pour introduire ledit filament dans la poche (4) à travers l'introducteur (6).

## Description

La présente invention concerne un dispositif destiné au traitement de lésions de la paroi de vaisseaux sanguins, en particulier les faux-anévrismes, par coagulation.

Une cause classique de formation des faux-anévrismes est la ponction sur un vaisseau sanguin. Il peut arriver qu'à la suite de telles ponctions, la paroi d'un vaisseau soit lésée et qu'une poche contenant du sang se forme à l'extérieur du vaisseau. Une telle poche est désignée en chirurgie comme un "faux-anévrisme", par analogie avec un anévrisme résultant de l'altération des parois d'un vaisseau. En outre, l'utilisation de produits anticoagulants (fréquente dans le cas d'interventions chirurgicales) favorise la formation des faux-anévrismes.

Une méthode connue pour éliminer la poche d'un faux-anévrisme consiste à refouler la poche par application de pression de l'extérieur ou à fermer le chenal d'alimentation de la poche par compression guidée. Une telle méthode n'est applicable que si la poche est aisément accessible, par exemple lorsque le faux-anévrisme se forme sur un côté du vaisseau opposé à la peau du patient, ou lorsque la pression est douloureuse ou inefficace.

Une autre méthode de traitement connue est de provoquer la coagulation du sang contenu dans la poche, ce qui a pour conséquence une nécrose de la dite poche.

Les tissus nécrosés sont alors éliminés naturellement par le système immunitaire, ne laissant subsister qu'une cicatrice minime sur la paroi du vaisseau.

Une injection directe de produits coagulants est malheureusement peu efficace et souvent contre-indiquée; ces produits risquent en effet d'être en grande partie entraînés par le flux sanguin, ce qui est particulièrement néfaste dans le cas de personnes sous traitement.

Une méthode à effet plus localisé consiste à insérer un filament en matériau biocompatible totalement inerte susceptible d'être abandonné in situ sans provoquer de réaction préjudiciable dans la poche de façon à y former une pelote.

Il s'agit toutefois d'une opération fastidieuse; pour que l'intervention soit efficace, il peut être nécessaire d'introduire plusieurs mètres de filament. Le temps nécessaire pour obtenir une coagulation suffisante est typiquement de une à deux heures.

Si l'on souhaite récupérer le filament, l'opération doit donc être répétée ou être purement et simplement interrompue pendant un laps de temps non négligeable, ce qui implique une infrastructure médicale importante.

Si le filament est abandonné en place, il peut interférer avec le bon déroulement d'interventions ultérieures pratiquées sur le même site ou à proximité de celui-ci, ce qui peut être particulièrement gênant dans le cas de maladies chroniques.

L'invention a pour but la mise au point d'un dispositif permettant de traiter les faux-anévrismes par coagulation en un temps très court compatible avec les délais d'opération, qui soit facile à manipuler et fiable.

A cet effet, l'invention a pour objet un dispositif pour provoquer la coagulation d'un faux-anévrisme, qui comprend un introducteur à valve et à aiguille amovible, un filament apte à être introduit en pelote dans une poche d'une lésion vasculaire en s'y repliant et un moyen d'introduction apte à introduire ledit filament dans la poche à travers de cet introducteur.

Suivant une première forme préférée, le filament est maintenu par son extrémité proximale de façon telle qu'il peut être retiré à la poche après un temps déterminé.

Le filament 18 est avantageusement constitué d'une pluralité de tronçons aboutés entre eux par des zones pliables.

Le filament 8, 18, 38 est de préférence creux et est percé longitudinalement d'une pluralité d'orifices 24 permettant d'instiller localement dans la poche 4 un produit coagulant.

Le filament 8, 28 peut comprendre un fil 30 en métal à mémoire de forme traité de façon telle qu'il présente une forme sensiblement rectiligne à température ambiante et une forme sensiblement enroulée à la température de l'organisme.

De façon avantageuse, le filament 28 comprend une âme 30 revêtue d'un entrelacement 32 en un matériau thrombogène (par exemple, une tresse).

Dans un autre mode de réalisation, le filament 8, 18, 28, 38 est apte à envoyer un courant électrique suffisant pour provoquer une électrocoagulation.

Suivant une autre forme avantageuse, le filament 8, 38 comprend une âme 40 cotressée avec un faisceau de fils 42 en un matériau thrombogène.

Le matériau de l'entrelacement 32, 42 est choisi avantageusement entre la soie naturelle et le Dacron®.

Dans une autre forme de réalisation, le filament 8 est en matériau à effet thrombogène bio-résorbable.

Le matériau du filament 8 a par exemple une période de résorption minimale de 20 minutes.

De façon préférée, le moyen 19 pour introduire le filament comprend une enveloppe 50 longitudinalement déchirable dans lequel est logé le filament 8, 18, 28, 38.

Le moyen 19 pour introduire le fil peut aussi comprendre un guide 54 le long duquel le filament 8, 18, 28, 38 est appliqué de manière détachable ou une tige 60 à extrémité en boucle 62 élastiquement déformable apte à introduire le filament 8, 18, 28, 38 dans la poche par un mouvement alternatif.

Le filament est de préférence radio-opaque.

L'usage du dispositif suivant l'invention permet de ramener le temps nécessaire à la coagulation à quelques minutes, ce qui permet de pratiquer une intervention pratiquement sans hiatus, bénéfique à la fois pour le praticien et le patient.

La longueur du filament est considérablement réduite par rapport à la méthode empirique décrite ci-dessus, ce qui réduit le temps d'introduction et les risques liés à l'opération.

Le filament ayant un effet totalement localisé à la poche de l'anévrisme, il n'est plus nécessaire de procéder à une injection de produits coagulants sous forme liquide, ces produits ne risquent donc plus d'être disséminés dans l'organisme.

L'expérimentation montre qu'un traitement faisant appel au dispositif selon l'invention est fiable car le faux-anévrisme ainsi coagulé présente un très faible risque de relyse, après retrait ou élimination du fil au bout d'un temps prédéterminé.

D'autres caractéristiques et avantages de l'invention apparaîtront plus clairement dans la description ci-après de plusieurs modes de réalisation particuliers, donnés à titre illustratif et non limitatif en faisant référence aux dessins schématiques annexés, dans lesquels:
la figure 1 est une vue générale d'un site d'anévrisme;
la figure 2 est une vue agrandie montrant un dispositif suivant l'invention lors de son introduction dans la poche d'un faux-anévrisme;

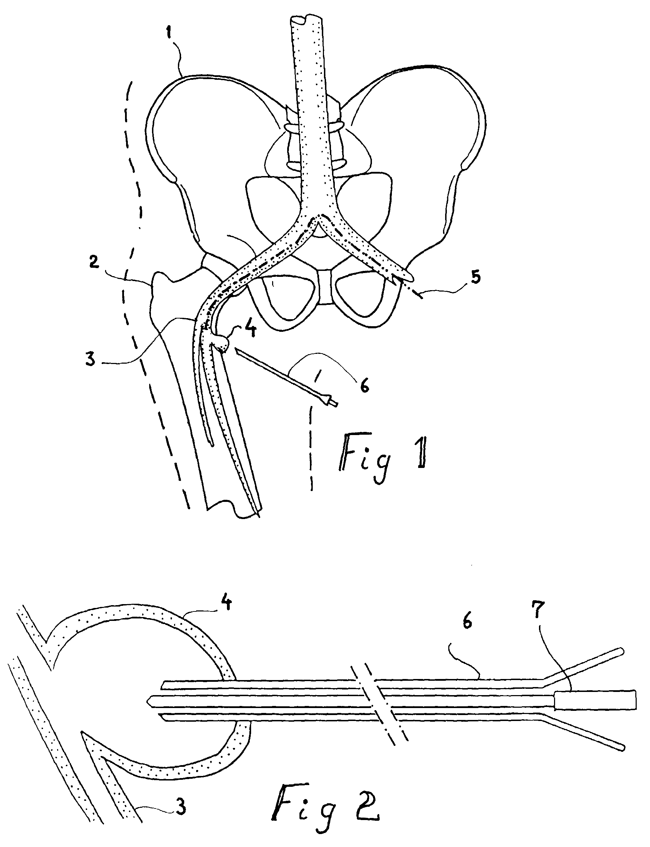

la figure 2 est une vue agrandie montant un dispositif suivant l'invention lors de son introduction dans la poche d'un faux-anévrisme;

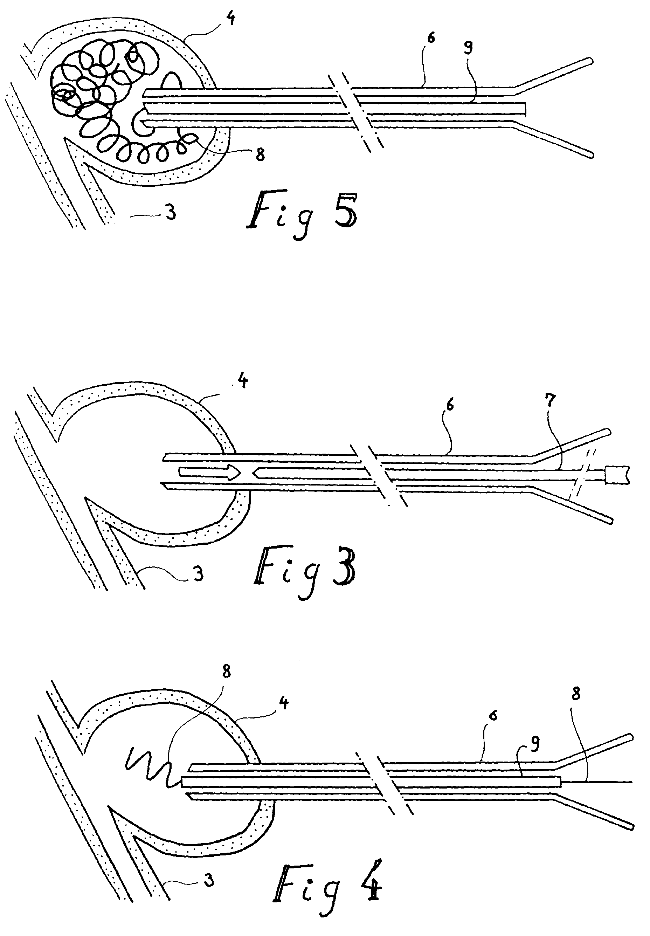

la figure 3 est une vue agrandie du dispositif de la figure 1, après retrait de l'aiguille de l'introducteur;
la figure 4 est une vue agrandie du même dispositif au moment de l'introduction d'un filament dans la poche;
la figure 5 est une vue analogue à la figure 4, montrant la poche du faux-anévrisme à la fin de la phase d'introduction;

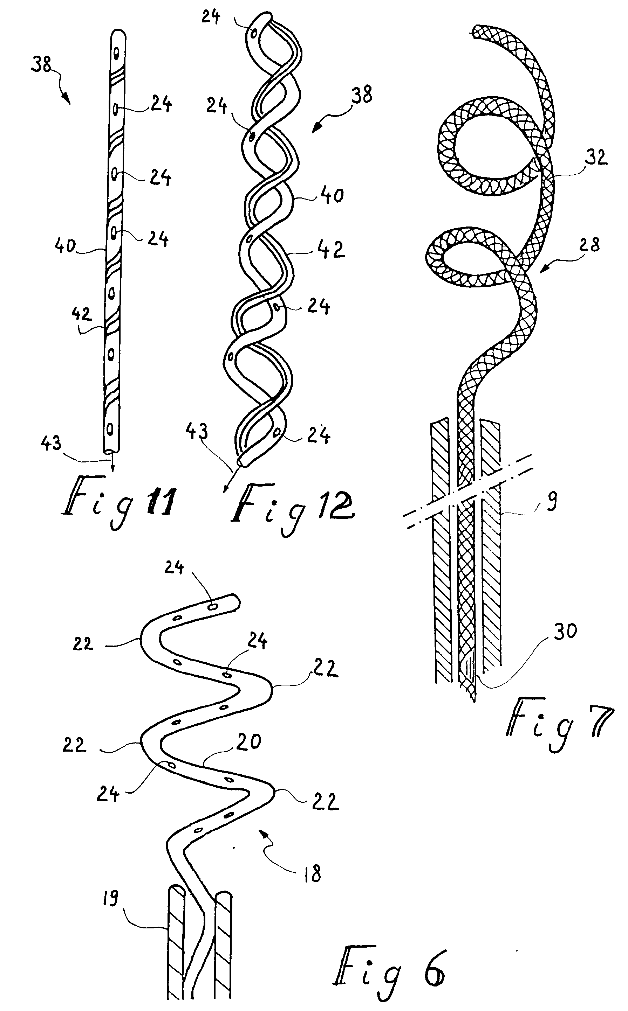

la figure 6 est une vue agrandie d'une forme de réalisation d'un dispositif suivant l'invention utilisant un cathéter creux repliable conforme à l'invention;

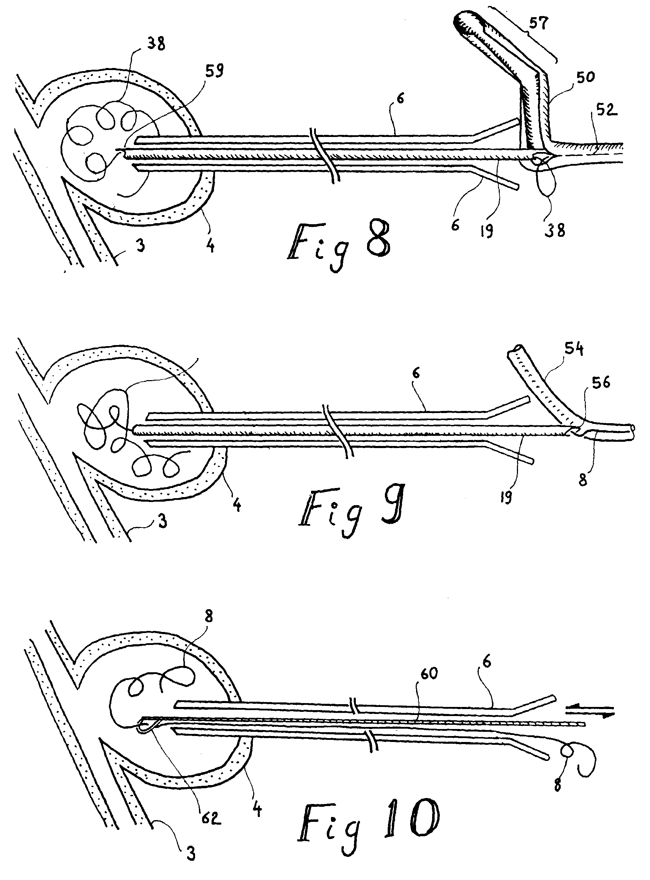

la figure 7 est une vue schématique d'une forme de réalisation impliquant un fil métallique à mémoire de forme;
la figure 8 est une vue agrandie d'une forme de réalisation, faisant appel à une gaine longitudinalement déchirable ou pelable;
la figure 9 est une vue analogue à la figure 6, dans laquelle le filament est collé de manière détachable sur l'extérieur d'un guide;
la figure 10 est une vue analogue à la figure 8, avec un moyen d'introduction à mouvement alternatif; et
les figures 11 et 12 sont des vues schématiques d'une autre forme de réalisation d'un filament suivant l'invention.

Sur la figure 1, sont représentés le bassin 1 d'un individu sur lequel s'articule un fémur 2, ainsi qu'une artère fémorale 3 présentant une poche 4 de faux-anévrisme. Un cathéter-guide 5 est inséré par voie controlatérale dans l'artère fémorale 3, de façon que son extrémité distale soit à proximité de la poche 4 du faux-anévrisme pour contrôler le point de ponction. La figure 2 montre l'introducteur 6 à valve d'un dispositif suivant l'invention dans lequel est logée une aiguille de ponction 7 piquée dans la poche 4. Pour faciliter la ponction du faux-anévrisme de la poche 4, l'introducteur 6 est de préférence muni d'un ou de plusieurs marqueurs radio-opaques (non représentés). L'introducteur 6 est sensiblement tubulaire et présente typiquement une longueur d'environ 10 cm et un diamètre extérieur variant de 1,3 à 2 mm.

Une fois que l'extrémité distale de l'introducteur 6 est introduite dans la poche 4, l'aiguille 7 peut être retirée, comme visible sur la figure 2. Un filament 8 présentant des caractéristiques qui seront décrites plus en détail ci-après peut être alors introduit à travers l'introducteur 6 dans la poche 4, par un moyen d'introduction 9 comme indiqué schématiquement sur la figure 4. Ce filament 8 présente la caractéristique de s'enrouler facilement sur lui-même en formant une sorte de pelote dans la poche 4, comme cela est visible sur la figure 5, ce qui a pour effet, en combinaison avec d'autres propriétés, décrites ci-après, de provoquer rapidement la coagulation du sang contenu dans la poche 4. Après un temps déterminé, de l'ordre de quelques minutes (au maximum de l'ordre de 20 minutes), le filament 8 ayant produit son effet sera éliminé comme décrit plus loin.

Un premier mode de réalisation d'un filament 18 suivant l'invention et de son moyen d'introduction 19 est montré à la figure 5. Le filament 18 est constitué d'une pluralité de tronçons 20 aboutés les uns aux autres par des zones pliables 22, chaque tronçon 20 ayant une longueur déterminée, par exemple de l'ordre de 5 à 10 mm, pour permettre un plissage en accordéon (ou en W) à l'intérieur de la poche 4 dès sa sortie de l'introducteur 6. Un tel filament 18 est réalisé de préférence en un matériau élastique, tel qu'un fil métallique ou plastique à effet ressort. Dans la forme de réalisation montrée à la figure 6, le filament 18 est en outre creux et présente une pluralité d'orifices 24 de façon à permettre une instillation localisée ou une injection de produit coagulant par l'extrémité proximale du filament 18, ce produit s'écoulant par les orifices 24 localement dans la poche 4 pour accélérer la coagulation du faux-anévrisme. L'extrémité proximale du filament 18 reste maintenue dans la partie proximale du dispositif. L'opérateur peut facilement contrôler un suintement éventuel de la poche 4 au cours du temps, avant de procéder au retrait du filament une fois que la coagulation est assurée. Le moyen d'introduction 19 est ici une gaine rigide qui, tout en laissant coulisser le filament 18, empêche tout repli intempestif de celui-ci avant sa sortie de l'introducteur 6.

La figure 7, montre une autre forme de réalisation 28 du filament 8 suivant l'invention; dans cette forme de réalisation, le filament comprend une âme 30 formée d'un fil métallique à mémoire de forme (tel que le Nitinol). Le filament 28 est introduit à travers l'introducteur 6 dans la poche 4, le long d'un tronçon de cathéter creux 9. Le fil métallique dont est formée l'âme 30 a subit un traitement thermique préalable lui fixant un seuil de transformation situé légèrement sous la température du corps humain; il présente une première forme sensiblement rectiligne à température ambiante (à l'extérieur du corps du patient); toutefois, dès que sa température dépasse le seuil de transition qui lui a été assigné, en l'occurrence, dès qu'il franchi la pointe de l'introducteur 6, il adapte aussitôt une forme recourbée et s'enroule à la manière d'un ressort à l'intérieur de la poche 4. L'extrémité proximale du filament 28 étant maintenue dans le dispositif à l'extérieur du corps, l'opérateur peut opérer son retrait après coagulation de la poche 4 au bout d'un temps déterminé. Au fur et à mesure de son retrait, le filament 28 reprend sa forme sensiblement rectiligne, ce qui réduit le frottement avec le cathéter. On peut combiner les deux modes de réalisation décrits ci-dessus en référence aux figures 6 et 7, le filament 8 présentant alors dans la poche 4 une allure grossièrement épicycloïdale.

La figure 7 montre une forme de réalisation du filament suivant l'invention associant à l'âme 30 une gaine tressée 32 en un matériau à action thrombogène.

Cette gaine 32 peut être réalisée notamment en fils de soie (e.a. de la soie d'araignée) ou en Dacron®. Les effets naturellement thrombogènes de ces matériaux sont ici exacerbés par la surface de contact importante développée dans le dispositif suivant l'invention. En outre, le matériau de la gaine peut être imprégné de différentes substances pharmacologiques à effet thrombogène. De telles substances peuvent également être intégrées ou greffées chimiquement dans la structure des fils tressés formant la gaine 32.

Suivant une autre forme de réalisation, les fils ne sont pas tressés autour d'une âme mais cotressés ou torsadés avec celle-ci. Dans le mode de réalisation représenté aux figures 11 et 12, l'âme 40, après introduction dans la poche 4 peut induire un relâchement du faisceau de fils 42 comme montré à la figure 12.

Dans ce mode de réalisation, l'âme 40 est également creuse, ce qui permet, comme à la figure 6, une instillation localisée de produits coagulants. L'âme 40 comprend aussi un moyen de rigidification en l'occurrence une tige mobile activable depuis l'extrémité proximale du filament.

Le moyen de guidage utilisé peut revêtir différentes formes: il peut s'agir, comme dans les modes de réalisation montrés dans les figures précédentes, d'un tronçon plus ou moins long de gaine rigide.

Les figures 8 à 10 montrent différentes variantes de ce moyen de guidage facilitant la manipulation de filaments 5, 18 suivant l'invention. On peut ainsi ici en conjonction avec un cathéter 9 utiliser une gaine longitudinale déchirable 50 (gaine dite "pelable") qui entoure le filament 8. Cette gaine, présentant une ou plusieurs lignes de déchirement longitudinales 52, se fend au fur et à mesure de l'introduction du filament 8 dans l'introducteur 6. Si le filament 8 présente une très faible rigidité, il peut également être appliqué de façon détachable par un adhésif sur la surface extérieure d'un tuteur-guide 54, notamment le long d'une rainure. Un moyen de séparation adéquat, tel qu'une arête biseautée, détache le filament de son tuteur 54 à son entrée dans l'introducteur 6 comme représenté à la figure 9.

Dans le mode de réalisation illustré sur la figure 8, le filament 38 est réalisé en un matériau bio-résorbable, ce qui lui permet de se résorber automatiquement au bout d'un temps de séjour déterminé à l'intérieur de la poche 4. Ce fil 38 est logé à l'intérieur d'une succession de tronçons 57 de cathéter longitudinalement déchirables 50.

La segmentation en tronçons 57 permet une manipulation aisée du filament 38 dont la longueur peut être facilement estimée en fonction du nombre de tronçons utilisés.

Le matériau utilisé étant bio-résorbable, il n'est pas nécessaire ici de retirer le filament, il suffit de le détacher du moyen d'introduction 19. En conséquence, un moyen de sectionnement (en l'occurrence une arête biseautée 59) est prévu à l'extrémité distale de l'introducteur 6. En effectuant une traction relative sur le moyen d'introduction 19, l'opérateur provoque un sectionnement du filament 38. L'opération peut être répétée plusieurs fois puisque le filament 38 peut être introduit sans inconvénient sous forme discontinue.

La figure 10 montre un autre moyen d'introduction du filament 8, constitué ici d'une tige métallique 60 pourvue, à son extrémité distale, d'une boucle déformable en forme de lasso 62.

L'extrémité distale d'un filament 8 de l'invention ayant été introduite dans cette boucle 62, il suffit d'insérer la tige 60 dans l'introducteur 6 et d'effectuer un mouvement de va-et-vient avec la tige métallique 60, qui entraîne le filament 8 lors de son mouvement aller et le relâche lors de son mouvement de retour.

Les différents moyens d'introduction décrits ci-dessus peuvent bien sûr être utilisés en combinaison avec la forme de filament 8, 18, 28, 38 la plus appropriée en fonction du cas à traiter (notamment, suivant les dimensions et la position de la poche 4 de faux-anévrisme).

## Revendications

1. Dispositif pour le traitement d'anévrisme par coagulation, caractérisé en ce qu'il comprend un introducteur (6) à valve et à aiguille amovible (7), un filament (8, 18, 28, 38) apte à être introduit dans la poche (4) (où il restera temporairement) de cet anévrisme et à s'y enrouler sur lui-même et un moyen (19) pour introduire ledit filament dans la poche (4) à travers l'introducteur (6).

2. Dispositif selon la revendication 1, caractérisé en ce que le filament (8, 18, 28, 38) est maintenu par son extrémité proximale de façon telle qu'il peut être retiré de la poche après un temps déterminé.

3. Dispositif suivant la revendication 2, caractérisé en ce que le filament (18) est constitué d'une pluralité de tronçons (20) aboutés entre eux par des zones pliables (22).

4. Dispositif suivant l'une quelconque des revendications 2 et 3, caractérisé en ce que le filament (8, 18, 38) est creux et est percé longitudinalement d'une pluralité d'orifices (24) permettant d'instiller localement dans la poche (4) un produit coagulant.

5. Dispositif suivant l'une quelconque des revendications 2 à 4, caractérisé en ce que le filament (8, 28) comprend un fil (30) en métal à mémoire de forme traité de façon telle qu'il présente une forme sensiblement rectiligne à température ambiante et une forme sensiblement enroulée à la température de l'organisme.

6. Dispositif suivant l'une quelconque des revendications 2 à 5, caractérisé en ce que le filament (28) comprend une âme (30) est revêtue d'un entrelac (32) en un matériau thrombogène.

7. Dispositif suivant la revendication 6 caractérisé en ce que le revêtement est une tresse (32).

8. Dispositif suivant l'une quelconque des revendications 2 à 5, caractérisé en ce que le filament (8, 38) comprend une âme (40) cotressée avec un faisceau de fils (42) en un matériau thrombogène.

9. Dispositif suivant l'une quelconque des revendications 6 à 8, caractérisé en ce que le matériau de l'entrelacement (32, 42) est choisi entre la soie naturelle et le Dacron®.

10. Dispositif selon la revendication 1, caractérisé en ce que le filament (8) est en matériau à effet thrombogène bio-résorbable.

11. Dispositif suivant la revendication 10, caractérisé en ce que le matériau du filament (8) a une période de résorption minimale de 20 minutes.

12. Dispositif suivant l'une quelconque des revendications précédentes, caractérisé en ce que le moyen (19) pour introduire le filament est une enveloppe (50) longitudinalement déchirable dans lequel est logé le filament (8, 18, 28, 38).

13. Dispositif suivant l'une quelconque des revendications 1 à 11, caractérisé en ce que le moyen (19) pour introduire le fil est un guide (54) le long duquel le filament (8, 18, 28, 38) est appliqué de manière détachable.

14. Dispositif suivant l'une quelconque des revendications 1 à 11, caractérisé en ce que le moyen (19) pour introduire le filament est une tige (60) à extrémité en boucle (62) élastiquement déformable apte à introduire le filament (8, 18, 28, 38) dans la poche par un mouvement alternatif.

15. Dispositif selon l'une quelconque des revendications précédentes, caractérisé en ce que le filament est radio-opaque.

16. Dispositif selon l'une quelconque des revendications précédentes, caractérisé en ce que le filament (8, 18, 28, 38) est apte à envoyer un courant électrique suffisant pour provoquer une électrocoagulation.
